# EUROPEAN PATENT APPLICATION

(11) **EP 3 653 694 A1**
(43) Date of publication of application: **20.05.2020**
(21) Application number: 18831537.8
(22) Date of filing: 25.06.2018
(51) Int. Cl.: C12M 1/00

(54) **FISH EGG PROCESSING DEVICE**

(30) Priority: 10.07.2017 JP 2017134435
(71) Applicant: Sinfonia Technology Co., Ltd., Tokyo 105-8564 (JP)
(72) Inventor: NAKANISHI, Akira, Tokyo 105-8564 (JP); YAMANAKA, Yuji, Tokyo 105-8564 (JP); NAKAI, Saori, Tokyo 105-8564 (JP)
(74) Representative: TBK
(86) International application number: PCT/JP2018/024029
(87) International publication number: WO 2019/012972

(57) **Abstract**

There is provided a fish egg processing apparatus capable of performing a predetermined processing on fertilized eggs with higher efficiency.

A gene injection device 1 which is a fish egg processing apparatus according to the present invention includes a processing water tank 8 which processes collected fish eggs e and a processing device for injecting a predetermined substance into the fish eggs e positioned in the processing water tank 8, wherein the processing water tank 8 includes: a region prior to processing 81 where the width dimension is set such that a plurality of the fish eggs e are not allowed to lie side by side and form a line in order to house the fish eggs e prior to processing; and a region after processing 82 for housing the fish eggs e on which a predetermined processing has been performed.

## Description

### TECHNICAL FIELD

The present invention relates to a fish egg processing apparatus for performing a predetermined processing mainly on fish eggs.

### BACKGROUND ART

It is known that the use of eggs of small-sized fish such as zebrafish is useful in the technical field in which target substances such as recombinant proteins are produced using genetic engineering techniques by injecting genes into fertilized eggs. For example, when the zebrafish are used to obtain a target substance, a gene solution (vector) needs to be precisely injected into a spherical fertilized egg having a diameter of 0.9 mm to 1.3 mm. There is a known a microinjection technique in which, in order to prevent damages to fertilized eggs, a gene solution is injected by penetrating the egg membrane of a fertilized egg with an extremely thin needle having a tip with a diameter of several to several dozens of micrometers, and inserting the needle tip into the embryo. In this field, microinjection work using a manipulator, or the like, to prevent hand shake due to manual work or manual operation is generally employed; however, it is difficult to process fertilized eggs in an amount required to acquire the practical quantity of target substances, and the accuracy and the stability of an injection process are also limited. Therefore, various attempts have been made to achieve automation as described in the following patent documents.

### CITATION LIST

### PATENT LITERATURE

Patent Document 1: Japanese Patent No. 5647005
Patent Document 2: Japanese Patent No. 5823112
Patent Document 3: Japanese Patent No. 5787432.

### SUMMARY OF THE INVENTION

### PROBLEM TO BE SOLVED BY THE INVENTION

However, egg membranes and embryos of fish eggs are different in specific gravity. Therefore, the embryos are eccentrically positioned against egg membranes in vertical and downward direction by the gravity. In a conventional method to arrange and inject eggs into a plate or the like, it is difficult to inject genes after appropriately observing the depth and state of inserted needles and the situation of gene injection by a visual contact from upper and lower side of fish eggs and determining the inserting depth of gene injection needle. Further, it is difficult to image or sense the inserting depth of the gene injection needle from the front-rear or the side of fish egg conveying direction while avoiding the impact from components which realize a conveying method of the fish egg. In addition, understanding the situation of inserted needles or the inserting condition of gene injection solution is also the same.

Furthermore, an extra fine gene injection needle capable of conducting a microinjection applies to a mode which is disposable for prevention of contamination. However, the needle length or attachment structure is not often created at precision level which does not affect the precision of needle injection depth into fish eggs simply by attaching the needle to the attachment, accordingly, there is difference and variability in each length of the needle. Therefore, it is required to create design and setting procedure in view of attachment alignment to device side or various parts accumulated tolerance after precisely determining the tip positions of a plurality of needles in an area of appropriately several dozens of micrometers. Thus, a previous adjustment of complicated procedure is required. Even for specific skilled operators, the operation is difficult and the time is needed.

Further, the above-described fertilized eggs frequently undergo cell division, for example, at intervals of about 30 minutes, and therefore, in order to rapidly introduce a gene and obtain a desired fertilized egg, it is intended that a fertilized egg is processed with higher efficiency. Specifically, the related arts disclosed in the above patent documents are based on the assumption of what is called batch processing in which the processing speed is restricted due to the use of a predetermined container, or the like, and therefore fertilized eggs are not processed in a state suitable for processing on a constant basis. Further, needless to say, it is an important element to efficiently collect the fertilized eggs after processing, and currently, the art to collect the fertilized eggs with higher efficiency than the related arts disclosed in the above patent documents is also required.

Specifically, it is vital to convey with higher efficiency and to collect fertilized eggs after processing with higher efficiency so that the above-described fertilized eggs are processed with higher efficiency and a predetermined processing is performed on the fertilized eggs.

The present invention has been made in view of the above problems and a main object thereof is to provide a fish egg processing apparatus capable of performing a predetermined processing on the fertilized eggs with higher efficiency.

### MEANS FOR SOLVING THE PROBLEM

The present invention takes the following measures in view of the above problems.

That is, a fish egg processing apparatus of the present invention comprising: a processing water tank for processing collected fish eggs and a processing device for injecting a predetermined substance into the fish eggs positioned in the processing water tank, wherein the processing water tank includes: a region prior to processing where the width dimension of the region prior to processing is set such that the fish eggs form a line in order to house the fish eggs prior to processing; and a region after processing for housing the fish eggs on which a predetermined processing has been performed.

With the above configuration, there can be provided the fish egg processing apparatus capable of performing a determined processing with higher efficiency on the fertilized eggs which have been collected and obtained from the predetermined species of the fish.

A configuration such that a predetermined processing is performed more certainly on the fish eggs, may be a configuration in that the processing device includes: a placing part capable of mounting the fish eggs; and an introducing pipe having a pipe shape capable of introducing a predetermined substance into the fish eggs mounted on the placing part in a predetermined timing.

Further, in order that the fish eggs may be processed with higher efficiency, it is desirable that the placing part has: a disc body having a disk shape; and a housing recess capable of housing the fish egg which is formed in the peripheral part of the disk body at a predetermined pitch. Accordingly, the fish eggs can be processed with high efficiency.

In order that workers may more certainly process the fish eggs, it is desirable to include the region prior to processing in which the fish eggs can be visually recognized from the side via the processing water tank. In other words, a member with which the region prior to processing is made up, may be configured to be transparent.

In addition, in order that the fish eggs after processing may be efficiently collected, it is desirable to have a collecting tank for collecting the fish eggs on which a predetermined processing has been performed. Accordingly, it is possible to easily collect the fish eggs only by drawing the collecting tank up.

Further, in order to further improve quality of the fish eggs after processing to be collected, it is desirable to have an ejecting unit for ejecting the fish eggs on which a predetermined processing could not be performed by the processing device. Accordingly, it is possible to obtain the fish eggs on which the processing has been accurately performed with high precision, and when the fish eggs which have passed through the ejecting unit are collected, it is possible to effectively prevent the fish eggs which could be processed from leaking to outside.

### EFFECT OF THE INVENTION

Thus, according to the present invention described above, it is possible to provide a fish egg processing apparatus capable of performing a predetermined processing on fertilized eggs with higher efficiency.

### BRIEF DESCRIPTION OF THE DRAWINGS

FIG. 1 is a functional block diagram according to a first embodiment of the present invention;
FIG. 2 is an entire configuration explanatory view according to the first embodiment of the present invention;
FIG. 3 is a schematic configuration explanatory view illustrating a collected-egg conveying unit according to the first embodiment of the present invention;
FIG. 4 is a schematic configuration explanatory view illustrating a unnecessary-material separation unit according to the first embodiment of the present invention;
FIG. 5 is a configuration explanatory view illustrating a gene injection unit and a processing water tank according to the first embodiment of the present invention;
FIG. 6 is a schematic planar view according to FIG. 5;
FIG. 7 is an operation explanatory view according to the first embodiment of the present invention.

### MODE FOR CARRYING OUT THE INVENTION

An embodiment of the present invention is explained below with reference to the drawings.

A gene injection device 1 which is an example of egg processing system or fish egg processing device according to the present embodiment is used to promptly introduce a predetermined gene into each of fertilized eggs, which are fish eggs e collected from a breeding water tank B. Then, in the fish egg e into which the predetermined gene has been injected, cell division is repeated and a protein derived from the base sequence of the introduced gene is synthesized. Then, the protein is collected, extracted, and purified in appropriate timing and is used for, for example, drug development study and mass production.

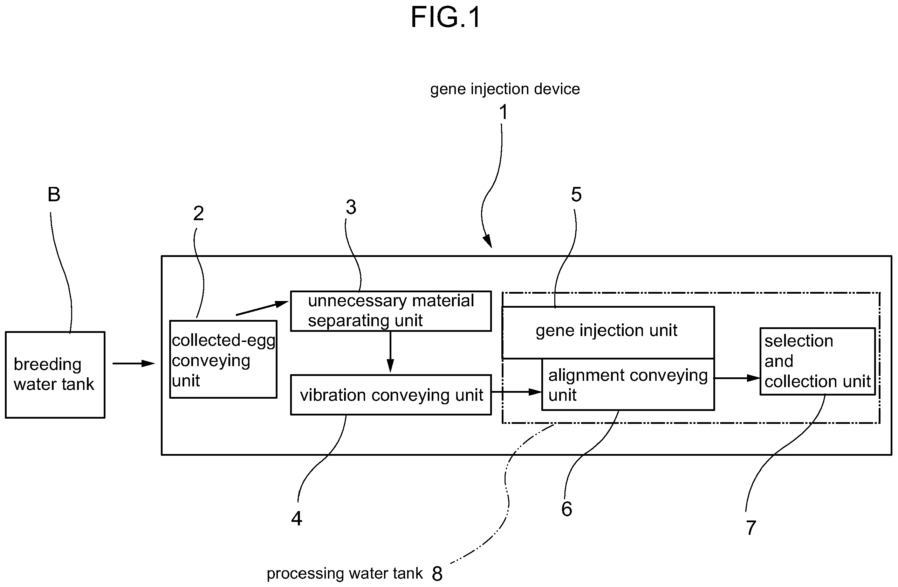

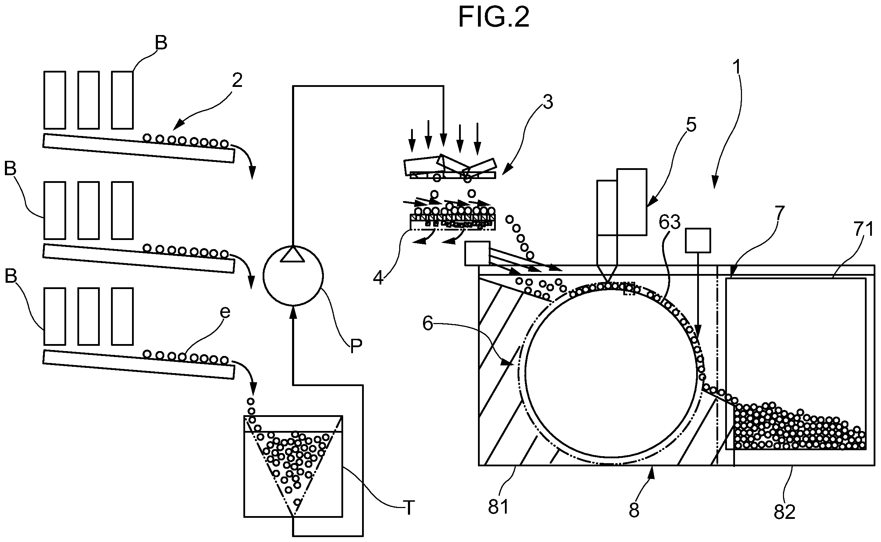

As illustrated in FIG. 1 and FIG.2, the gene injection device 1 includes: a collected-egg conveying unit 2 that is continuous to the breeding water tank B; an unnecessary-material separating unit 3 that separates the conveyed fish eggs e from unnecessary materials bg, sg such as breeding water w, excrement, or remaining food; a vibration conveying unit 4 that conveys, mainly by vibration, the fish eggs e separated from the unnecessary materials bg, sg; an alignment conveying unit 6 that conveys the fish eggs e conveyed by a vibration conveying unit 4, while arranging the fish eggs e in a predetermined state; a gene injection unit 5 that injects the predetermined gene into each of the fish eggs e conveyed by the alignment conveying unit 6; and a selection and collection unit 7 for efficiently collecting the fish egg e into which the predetermined gene has been injected. Further, according to the present embodiment, the gene injection unit 5 which is a processing device for performing a predetermined processing on the fish eggs e, the alignment conveying unit 6 and the selection and collection unit 7 which is a collection device are disposed in a processing water tank 8 having, in a planar view, substantially a T-shape.

Further, according to the present embodiment, a zebrafish egg having substantially a spherical shape with a diameter of approximately 1 mm is used as an example of the fish egg e. It is noted that, since fish is a vertebrate, a protein in the form that can be used for drug development is easily obtained by gene introduction, and in particular, zebrafish is known as the species with which the fish eggs e, which are fertilized eggs, are efficiently obtained from the breeding water tank B.

The configuration of each part of the gene injection device 1 is described below.

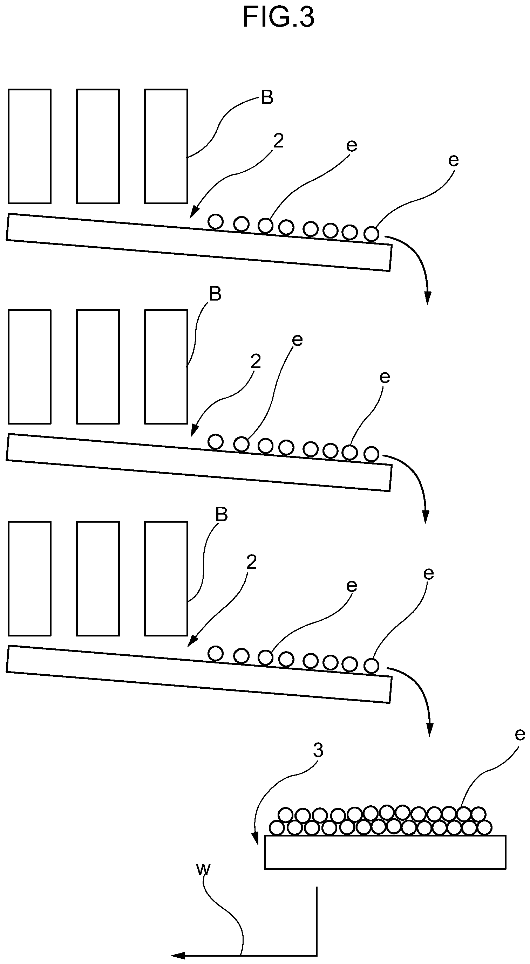

As illustrated in FIG.2 and FIG.3, the collected-egg conveying unit 2 is, for example, a water-gutter shaped passage configured to have a sloped shape so as to efficiently collect the fish eggs e from the breeding water tanks B arranged in plurality in parallel and at multiple stages in a vertical direction. As well as the fish eggs e, the collected-egg conveying unit 2 simultaneously conveys the breeding water w in which the fishes are bred, from the breeding water tanks B, and the fish eggs e and the breeding water w are once housed in a tank T and then lifted by a pump P and guided to the unnecessary-material separating unit 3. Specifically, the fish eggs e and the breeding water w are dropped down from the upper side and guided to the unnecessary-material separating unit 3 in a state where dropping energy is given. The tank T serves as a temporary cushion for collected and delivered fish eggs e and breeding water w, and in the example illustrated, for example, when spiral water current water is produced inside of the tank, water can be supplied with the pump P without any delay. Further, as a specific mode, damage or wear and tear due to an idle feeding with the pump P is prevented by detecting the lower limit of water level in the tank T, and an overflow detection is conducted by detecting the upper limit of water level. When overflow is generated in practice, the breeding water w is moved via a pipe (not illustrated) to the breeding water tank B. It is noted that, when the breeding water tank B can be placed at a sufficiently high position and the fish eggs e can be dropped freely from the breeding tank B to the unnecessary-material separating unit 3, the tank T and the pump P may be omitted.

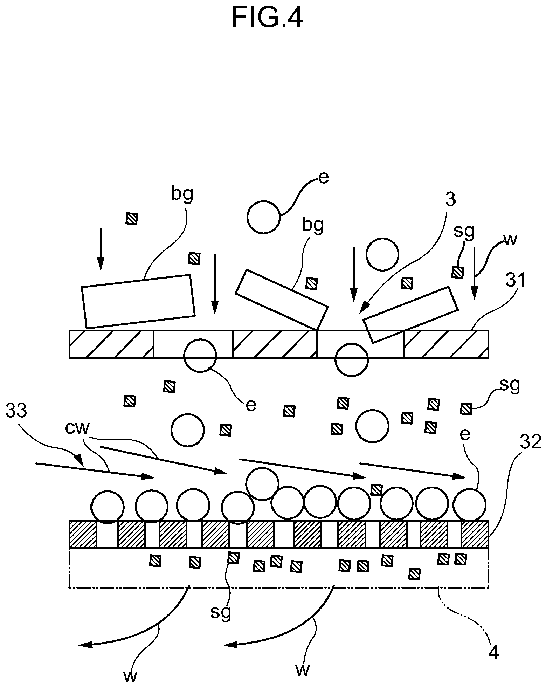

As specifically illustrated in FIG.4, the unnecessary-material separating unit 3 includes: a first net device 31 that allows the fish eggs e to pass therethrough and collects and removes the unnecessary materials bg larger than the fish eggs e; a second net device 32 that is supported by the vibration conveying unit 4 and has a mesh that does not allow the fish eggs e to pass therethrough; and a clean-water ejection method 33 that ejects clean water cw to the fish eggs e in the second net device 32. According to the present embodiment, applied is the configuration in that the breeding water w which passed through the second net device 32, or were crushed by colliding with the second net device 32 is further separated from, for example, the small unnecessary materials bg smaller than the fish eggs e which have been passed through the second net device 32, and the breeding water w is introduced to the breeding water tank B again. Further, according to the present embodiment, the clean-water ejection method 33 is used for prompt conveying the fish eggs e by ejecting the clean water cw to the fish eggs e along the direction in which the fish eggs e are conveyed by the vibration conveying unit 4. That is, according to the present embodiment, applied is the configuration that is not allowed the breeding water w, that has passed through the second net device 32 and that is potentially mixed by a bacteria, microorganism, or the like, to be introduced into the processing device disposed in the processing tank 8. Here, the clean water cw according to the present embodiment is not only distilled water or tap water but also water having less contamination than the breeding water w.

The vibration conveying unit 4 is intended to guide the fish eggs e to the processing water tank 8 by applying a predetermined vibration to the second net device 32. The fish eggs e to which the vibration has been applied by the vibration conveying unit 4 are conveyed and introduced into the processing water tank 8 quickly and efficiently.

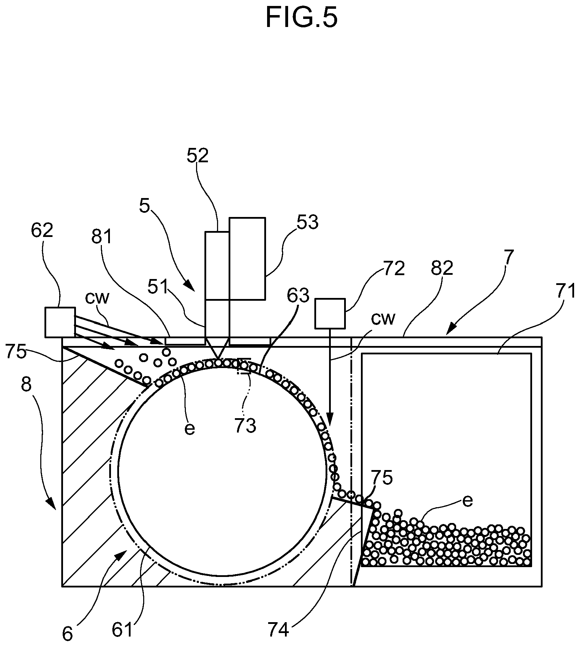

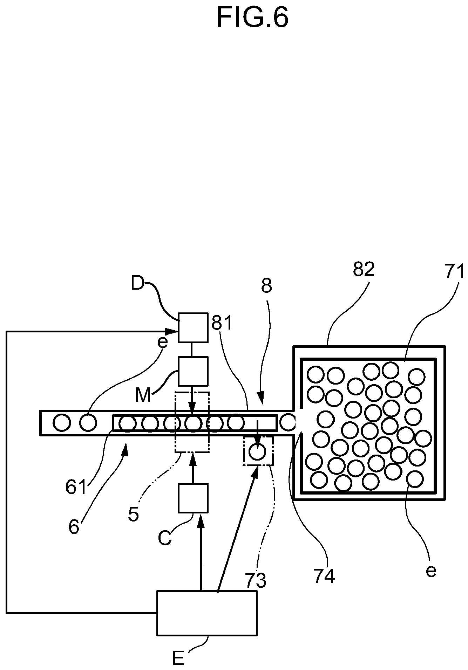

Here, as illustrated in FIG. 5 and FIG.6, according to the present embodiment, the gene injection unit 5, the alignment conveying unit 6 and the selection and the collection unit 7 are provided in the processing water tank 8. Specifically, the gene injection unit 5 is disposed above the processing water tank 8, and the alignment conveying unit 6 and the selection and the collection unit 7 are disposed inside of the processing water tank 8. Further, as illustrated in FIG. 2 and FIG.5, water is filled with up to the vicinity of the upper end of the processing water tank 8.

The processing water tank 8 is intended to process the collected fish egg e, and is provided with the gene injection unit 5 which is a processing device for injecting a predetermined substance into fish eggs e, and the alignment conveying unit 6. Specifically, the processing water tank 8 includes: the region prior to processing 81 where the inner thickness dimension is set such that a plurality of fish eggs e are not allowed to lie side by side in order to house the fish eggs e; and the region after processing 82 for housing the fish eggs e on which predetermined processing has been performed. For further details, the processing water tank 8 has, in a planar view, substantially a T-shape in which the region prior to processing 81 where the inner thickness dimension is set such that a plurality of fish eggs e are not allowed to lie side by side, is continuous to the region after processing 82. Further, according to the present embodiment, at least, in order that the inside of the region prior to processing 81 may be visually recognized, at least, the region prior to processing 81 is made up with a transparent material.

The alignment conveying unit 6 is intended to line up the fish eggs e in order to allow the fish eggs e that have been introduced into the region prior to processing 81 to be easily processed by the gene injection unit 5. The alignment conveying unit 6 has; an alignment board 61 which is a disk body having a disk shape and disposed with a plurality of recesses 63 which are housing recesses formed in the peripheral part of the disk body at a predetermined pitch; and an alignment pump 62 for ejecting the clean water cw toward the alignment board 61. The clean water cw ejected from the alignment pump 62 allows the fish eggs e that have been introduced into the region prior to processing 81 to be smoothly guided to the alignment board 61, and then, the position of each of the fish eggs e is determined in the recess 63. The alignment board 61 corresponds to a placing part capable of mounting the fish eggs e. Then, according to the present embodiment, as illustrated in FIG.6, rotation of the alignment board 61 is precisely controlled by activating a AC servomotor M through a motor driver D. The motor driver D is controlled by a controller E.

The gene injection 5 is intended to inject a predetermined gene into each of the fish eggs e conveyed by the alignment conveying unit 6 and includes; a capillary 51 having a substantially needle shape for directly injecting the gene into each of the fish eggs e; a syringe pump 52 for supplying a predetermined amount of gene into the capillary 51; and a positioner 53 for determining the vertical position of the capillary 51 and the syringe pump 52. Then, the capillary 51 corresponds to an introducing pipe having a pipe shape capable of introducing a predetermined substance into the fish eggs e in a predetermined timing.

In addition, according to the present embodiment, the gene injection device 1 includes a selection and collection unit 7 for efficiently collecting the fish eggs e into which the gene has been injected from the alignment conveying unit 6 to the region after processing 82.

The selection and collection unit 7 corresponds to a collection device or a collection tank for collecting fish eggs e after processing. The selection and collection unit 7 includes: a collection container 71 which is the collection tank housed in the region after processing 82; a collection pump 72 that is provided in the region prior to processing 81 in the vicinity of the region after processing 82 and intended to eject the clean water cw toward the alignment board 61; an ejecting unit 73 for ejecting the fish eggs e into which the gene was not able to be accurately introduced by the gene injection unit 5 that constitute a processing device out of a processing tank 8; and a guide 75 that is provided such that the lower half of the alignment board 61 is covered in the processing tank 8 and intended to guide the fish eggs e from the region prior to processing 81 to the region after processing 82. Then, the collection container 71 according to the present embodiment is provided with a slit 74 at the part on the region prior to processing 81. Whereby, it is possible that the collection container 71 efficiently introduces the fish eggs e that have moved from the region prior to processing 81 into the collection container 71. Further, according to the present embodiment, a part of the guide 75 forms so as to enter into the collection container 71, whereby, the fish eggs e can be efficiently guided to the collection container 71.

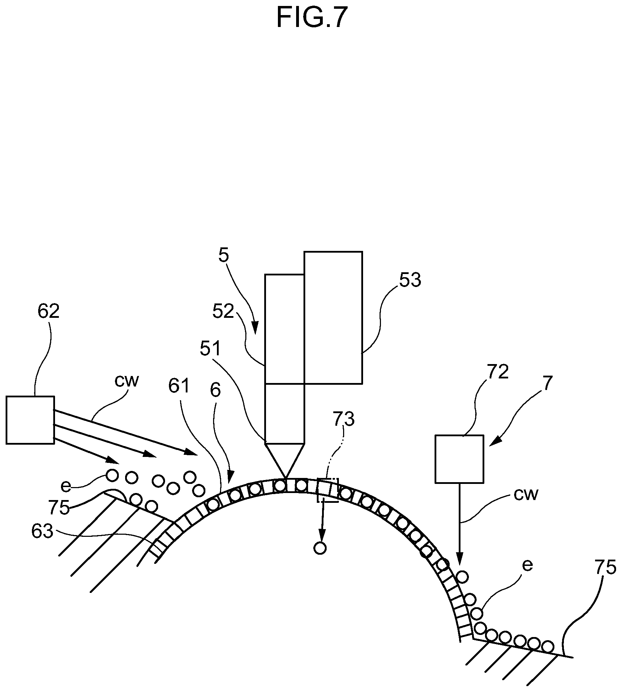

Here, according to the present embodiment, as illustrated in FIG.5, FIG,6 and FIG 7, applied is the configuration such that the fish eggs e that have been introduced into the processing tank 8 are processed with higher efficiency. That is, the fish eggs e that have been introduced into the region prior to processing 81 in the processing tank 8 are smoothly guided to the recess 63 by the clean water cw ejected from the alignment pump 62. Then, the fish eggs e that have been each housed into the recess 63 move up to the place just under the capillary 51 without difference in order by rotation of the alignment board 61. Here, the thickness of the region prior to processing 81 is set to a width such that two or more fish eggs e are not allowed to lie side by side, and the region prior to processing 81 is made up with a transparent member. That is, the fish eggs e that have been guided up to the place just under the capillary 51 is surely in a state such that each of the fish eggs e can clearly have a visual recognition one by one. Then, the capillary 51 is disposed in a state where the position of inner embryo of each of the fish eggs e can clearly have a visual recognition with a camera C illustrated in FIG.6. Therefore, the gene can be accurately injected into the embryo while the camera C and the gene injection device 5 are precisely controlled. Here, since the alignment board 61 is configured to have a circular shape and the recess 63 is placed at the same interval, the controller E allows the recess 63 to be accurately positioned at the place just under the capillary 51. Here, if the camera C can visually recognize that the gene cannot be accurately injected into the fish eggs e, the fish eggs e are allowed to pass through the ejecting unit 73 provided at the place where the alignment board 61 is rotated at a predetermined angle and to be collected separately. Accordingly, the fish eggs e that have potentially been recombined are not allowed to leak to outside unnecessarily. In addition, the fish eggs e into which the gene has been accurately injected in a high ratio are guided to the region after processing 82. Furthermore, the fish eggs e into which the gene has been injected by the capillary 51 are certainly separated from the recess 63 by the clear water cw ejected from the collection pump 72. Then, the fish eggs e are smoothly guided to the region after processing 82, pass through the slit 74, and are housed into the collection container 71.

As described above, the gene injection device 1 which is the fish egg processing apparatus according to the present embodiment is characterized by including; the processing tank 8 for processing the fish eggs e collected, and the processing device for injecting a predetermined substance into the fish eggs e positioned in the processing tank 8, wherein the processing tank 8 includes: a region prior to processing 81 where the width dimension set such that a plurality of the fish eggs e are not allowed to lie side by side and form a line in order to house the fish eggs e prior to processing; and a region after processing 82 for housing the fish eggs e on which a predetermined processing has been performed.

With the configuration, realized is the gene injection device 1 capable of performing a predetermined processing on the fertilized eggs collected and obtained from a predetermined species of fish with higher efficiency.

As a configuration such that a predetermined processing is more certainly performed on the fish eggs e, according to the present embodiment, applied is the configuration in that the processing device includes, the alignment conveying unit 6 which is a placing part on which the fish eggs e can be mounted; and the capillary 51 which is a introducing pipe having a pipe shape that allows a predetermined substance to be introduced in a predetermined timing into the fish eggs e mounted on the alignment conveying unit 6.

Further, in order that the fish eggs e may be processed with higher efficiency, according to the present embodiment, the alignment conveying unit 6 is configure to have; the alignment board 61 which is a disk body having a disk shape; and the recess 63 capable of housing the fish egg e which is formed in the peripheral part of the disk body 61 at a predetermined pitch.

Then, in order that operators may process on the fish eggs e more certainly, according to the present embodiment, in order to configure the processing tank 8 in the region prior to processing 81 capable of being visually recognized from the side, specifically, a material with which the region prior to processing 81 is made up, is configure to be transparent. Accordingly, the inserting depth of the capillary 51 into the fish eggs e, housing condition of the fish eggs e into the recess 63, and also, in many cases, injecting position of the gene for injection prospectively colored or the distribution can be clearly imaged with the camera C from the side. Further, for example, when an image processing method or the like is separately used, a series of these processing is entirely automized easily. Relative position of the capillary 51 to the fish eggs e can be appropriately adjusted. Therefore, this does not only allow to effectively prevent damage due to a wrong operation of the capillary 51, but also contributes to creation of system that allows to effectively improve a production efficiency of a target substance derived from the injected gene due to an accurate operation of the capillary 51.

In addition, in order that the fish eggs e after processing may be efficiently collected, according to the present embodiment, provided is the collection container 71 which is a collection tank for collecting the fish eggs e on which a predetermined processing has been performed.

Additionally, in order to further improve quality of the fish eggs e after processing to be collected, according to the present embodiment, provided is the ejecting unit 73 for ejecting the fish eggs e on which a predetermined processing could not been performed by the processing device.

Although the embodiment of the present invention has been described above, the present invention is not limited to the configuration according to the above-described embodiment. Although the mode for introducing a gene into a fish egg is disclosed in the above embodiment, the mode for injecting different substances from genes, e.g., cells such as human cancer cells, drugs, drug candidate substances, chemical substances such as toxic substances, or food additives such as seasonings or coloring agents into a fish egg is not prevented. In addition, according to the above embodiment, although a zebrafish egg is used as a fish egg, it is obviously possible to use another kind of fish egg. Further, as well as the specific arrangement of individual components, the detailed mode such as specific water flow in the processing water tank or other configurations can be variously modified within the scope of the present invention.

### INDUSTRIAL APPLICABILITY

The present invention is available as a fish egg processing apparatus for performing a predetermined processing mainly on fish eggs.

### DESCRIPTION OF REFERENCE NUMERALS

- 1: gene injection device
- 2: collected-egg conveying unit
- 3: unnecessary-material separating unit
- 4: vibration conveying unit
- 5: gene injection unit
- 6: alignment conveying unit
- 7: selection and collection unit
- 8: processing water tank
- B: breeding water tank
- e: fish egg
- cw: clean water
- w: breeding water

## Claims

1. A fish egg processing apparatus comprising:
a processing water tank for processing collected fish eggs; and
a processing device for injecting a predetermined substance into the fish eggs positioned in the processing water tank,
wherein the processing water tank includes: a region prior to processing where the width dimension of the region prior to processing is set such that the fish eggs form a line in order to house the fish eggs prior to processing; and a region after processing for housing the fish eggs on which a predetermined processing has been performed.

2. The fish egg processing apparatus according to claim 1, wherein the processing device includes: a placing part capable of mounting the fish eggs; and an introducing pipe having a pipe shape capable of introducing a predetermined substance into the fish eggs mounted on the placing part in a predetermined timing.

3. The fish egg processing apparatus according to claim 2, wherein the placing part has: a disc body having a disk shape; and a housing recess capable of housing the fish egg which is formed in the peripheral part of the disk body at a predetermined pitch.

4. The fish egg processing apparatus according to claim 1 including the region prior to processing in which the fish eggs can be visually recognized from the side via the processing water tank.

5. The fish egg processing apparatus according to claim 2 including the region prior to processing in which the fish eggs can be visually recognized from the side via the processing water tank.

6. The fish egg processing apparatus according to claim 3 including the region prior to processing in which the fish eggs can be visually recognized from the side via the processing water tank.
